(19) Europäisches Patentamt
European Patent Office
Office européen des brevets

(11) Veröffentlichungsnummer: **0 009 161**
**B1**

(12) # EUROPÄISCHE PATENTSCHRIFT

(45) Veröffentlichungstag der Patentschrift: **19.05.82**

(21) Anmeldenummer: **79103258.4**

(22) Anmeldetag: **04.09.79**

(51) Int. Cl.³: **C 08 G 16/02,**
C 07 D 211/74, C 08 K 5/34

(54) **Polyalkylpiperidin-Formaldehyd-Polymerisate, Verfahren zu ihrer Herstellung und ihre Verwendung.**

(30) Priorität: **13.09.78 DE 2839711**

(43) Veröffentlichungstag der Anmeldung:
**02.04.80 Patentblatt 80/7**

(45) Bekanntmachung des Hinweises auf die Patenterteilung:
**19.05.82 Patentblatt 82/20**

(84) Benannte Vertragsstaaten:
**CH DE FR GB IT NL**

(56) Entgegenhaltungen:
DE - A - 2 719 131
DE - A - 2 731 378
SU - A - 475 362
US - A - 2 540 886

HOUBEN-WEYL "Methoden der organischen Chemie" 4. Auflage, Band XIV/2 1963, GEORG THIEME VERLAG, Stuttgart, Seiten 416 bis 423

(73) Patentinhaber: **HOECHST AKTIENGESELLSCHAFT**
**Postfach 80 03 20**
**D-6230 Frankfurt/Main 80 (DE)**

(72) Erfinder: **Wiezer, Hartmut, Dr.**
**Hans-Fischer-Strasse 6**
**D-8906 Gersthofen (DE)**
Erfinder: **Korbanka, Helmut, Dr.**
**Birkenstrasse 24**
**D-8901 Adelsried (DE)**
Erfinder: **Pfahler, Gerhard, Dr.**
**Karlsbader Strasse 27**
**D-8900 Augsburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 009 161 B1

# Polyalkylpiperidin-Formaldehyd-Polymerisate, Verfahren zu ihrer Herstellung und ihre Verwendung

Es ist bekannt, daß Polyalkylpiperidone-4 der Formel (I), in denen $R^2 = R^3 = H$ und $R^4 = CH_3$ ist, mit der 2- bis 4fach molaren Menge wäßrigen Formaldehyds unter schwach basischen Bedingungen bei niedrigen Temperaturen zu einer Spiroverbindung kondensieren, die hydriert werden kann (DE—A 27 31 378 und darin zitierte Sekundärliteratur, z.B. SU—A 475 362). Die Verbindungen sollen als Stabilisatoren für organisches Material brauchbar sein, über die Stabilisator-Wirksamkeit ist jedoch nichts vermerkt.

Überraschenderweise wurde gefunden, daß durch Reduzierung der Formaldehydmenge bei sonst ähnlichen Reaktionsbedingungen jedoch in stärker basischem Medium völlig andere Reaktionsprodukte, und zwar Polymere aus 2 bis ca. 200 Polyalkylpiperidoneinheiten erhalten werden können, und diese hervorragend wirksame Lichtstabilisatoren mit unerwartet geringer Flüchtigkeit und hoher Extraktionsfestigkeit darstellen.

Diese Kombination erwünschter Eigenschaften ließ sich nicht voraussehen. So wird z.B. in der DE—A 27 19 131 die Verwendung polymerer piperidinverbindungen als Lichtschutzmittel mit geringer Flüchtigkeit und hoher Extraktionsfestigkeit beschrieben, es zeigt sich jedoch, daß im Vergleich zu niedrigmolekularen Produkten ähnlicher Strukturmerkmale mit der Steigerung des Molgewichts ein Nachlassen der Wirksamkeit einhergehen kann. Beispielsweise stabilisiert das Bis-(tetramethyl-4-piperidin-4-yl)-sebazat Polypropylen besser als das Oligomere das Beispiels 1 der DE—A 27 19 131.

Die Erfindung betrifft somit ein Verfahren zur Herstellung von bisher nicht bekannten Polyalkylpiperidin-Formaldehyd-Polymerisaten, die neuen Polymeren selbst sowie die Verwendung derselben als Lichtschutzmittel für synthetische Polymermassen.

Dieses Verfahren zur Herstellung von Polyalkylpiperidin-Formaldehyd-Polymerisaten durch Umsetzen eines Polyalkylpiperidons-4 der allgemeinen Formel (I)

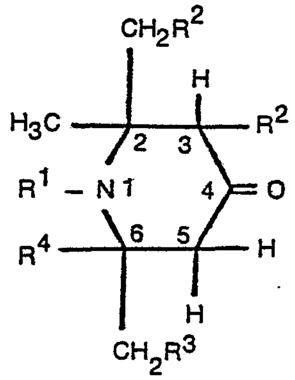

(I)

in welcher

$R^1$ Wasserstoff, Sauerstoff, $C_1$- bis $C_{12}$-Alkyl, vorzugsweise Wasserstoff, Sauerstoff oder $C_1$- bis $C_4$-Alkyl und insbesondere Wasserstoff ist,

$R^2$ und $R^3$ entweder gleich sind und Wasserstoff oder eine $C_1$- bis $C_5$-Alkylgruppe, vorzugsweise Wasserstoff oder eine Methylgruppe und insbesondere Wasserstoff bedeuten, wobei dann

$R^4$ eine Methylgruppe ist, oder auch

$R^2$ Wasserstoff oder $C_1$- bis $C_5$-Alkyl, vorzugsweise Wasserstoff oder Methyl und insbesondere Wasserstoff ist, und

$R^3$ und $R^4$ zusammen mit den an sie gebundenen Kohlenstoffatomen eine $C_5$- oder $C_6$-Cycloalkylgruppe oder eine Gruppe der Formel

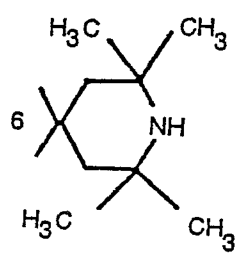

bedeuten,

mit Paraformaldehyd in Gegenwart eines alkalischen Katalysators und eines aliphatischen $C_1$- bis $C_{10}$-Monoalkohols bei erhöhten Temperaturen, wobei die Umsetzungsprodukte nach an sich bekannten Verfahren auch noch vollständig oder teilweise hydriert werden können, ist dadurch gekennzeichnet, daß man die 0,5- bis 1,1fach molare Menge Paraformaldehyd, bezogen auf Polyalkylpiperidon, bei Anwesenheit einer Alkalibase einsetzt und bei 50 bis 180°C arbeitet.

Besonders bevorzugt sind die Polymerisate, die auf der Basis von 2,2,6,6-Tetramethylpiperidon-4 hergestellt werden.

Dieser Reaktionsverlauf war nicht zu erwarten; man hätte eher damit rechnen müssen, daß sich auch hier die literaturbekannten Spiroverbindungen, allerdings mit geringeren Ausbeuten, bilden würden. Daß dies nicht der Fall ist, konnte keineswegs vorhergesehen werden, ebensowenig, daß es möglich sei, die extrem sterisch gehinderten Ausgangsketone mit Formaldehyd zu polykondensieren, so wie es für die Polykondensation von sterisch nicht gehinderten Keton-Methylengruppen in Houben-Weyl Bd. XIV/2, Seite 418, sowie der US—A 25 40 886 beschrieben ist.

Die neuen Polyalkylpiperidinpolymerisate lassen sich durch die allgemeine Strukturformel (II) beschreiben.

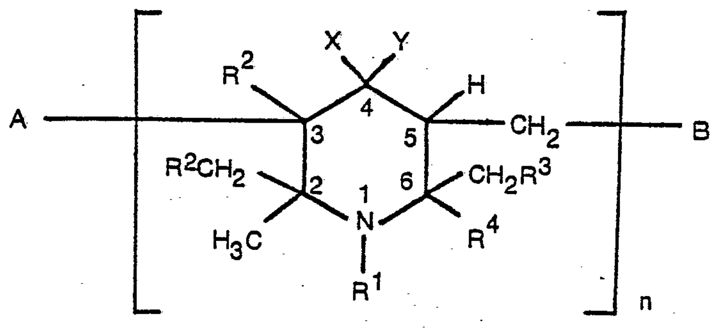

(II)

X und Y haben zusammen die Bedeutung von Sauerstoff oder sind verschieden und bedeuten dann Wasserstoff oder eine Hydroxylgruppe.

$R^1$ ist Wasserstoff, Sauerstoff oder $C_1$- bis $C_{12}$-Alkyl, vorzugsweise Wasserstoff, Sauerstoff oder $C_1$- bis $C_4$-Alkyl und insbesondere Wasserstoff,

$R^2$ und $R^3$ sind entweder gleich und stehen für Wasserstoff oder eine $C_1$- bis $C_5$-Alkylgruppe, vorzugsweise für Wasserstoff oder eine Methylgruppe und insbesondere für Wasserstoff, wobei dann $R^4$ eine Methylgruppe ist.

$R^2$ kann schließlich Wasserstoff oder $C_1$- bis $C_5$-Alkyl, vorzugsweise Wasserstoff oder Methyl sein, und

$R^3$ und $R^4$ zusammen mit den an sie gebundenen Kohlenstoffatomen eine $C_5$- oder $C_6$-Cycloalkylgruppe oder eine Gruppe der Formel

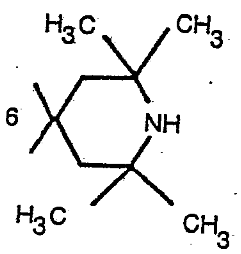

bilden.

Die Endgruppe

A ist Wasserstoff oder eine Hydroxymethylengruppe.

A und $R^2$ können, sofern $R^2$ = Wasserstoff ist, eine Methylengruppe bilden, wenn sich aus A = Hydroxymethylen mit $R^2$ Wasser abspaltet.

B ist ebenfalls eine Endgruppe und steht für Wasserstoff oder eine Hydroxygruppe oder eine Gruppe der Formel (III)

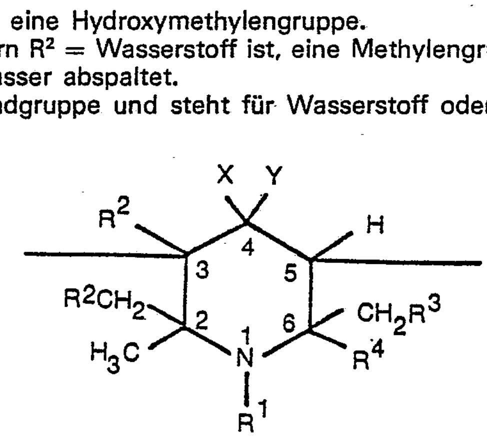

(III)

worin $R^1$ bis $R^4$ und X und Y die angegebenen Bedeutungen haben. Die Verknüpfung kann über das C-Atom 3 oder 5 erfolgen; die freibleibende Bindung ist dann durch H abgesättigt.

Wenn B eine Hydroxylgruppe ist, kann sich durch Abspaltung von Wasser eine $CH_2$-Gruppe bilden, die mit einer Doppelbindung an den Polyalkylpiperidinring gebunden ist.

n ist eine Zahl von 2 bis 200, vorzugsweise 2 bis 70 und insbesondere 2 bis 30.

Die Polyalkylpiperidinpolymerisate der Struktur (II), in denen X und Y zusammen Sauerstoff bedeuten, werden durch Kondensation von jeweils 1 Mol einer Verbindung gemäß Formel (I) mit der 0,5- bis 1,1fach molaren Menge Paraformaldehyd unter Verwendung katalytischer Mengen von Alkalibasen hergestellt. Man geht hierbei so vor, daß man der Polyalkylpiperidon der Formel (I) in der 0,2- bis 0-fachen, vorzugsweise der 0,5- bis 5fachen und insbesondere der 0,5- bis 2fachen Menge eines $C_1$- bis $C_{10}$-Alkohols vorlegt und eine Lösung von Paraformaldehyd in der 3- bis 10fachen, vorzugsweise 5- bis 10fachen, und insbesondere 5- bis 8fachen Gewichtsmenge desselben Alkohols, dem 1 bis 10, vorzugsweise 1 bis 5 Gew.-%, bezogen auf Polyalkylpiperidon der Formel (I), der Alkalibase als Katalysator zugesetzt sind, auf einmal oder auch im Verlaufe von bis zu 5 Stunden zugibt. Die Reaktionstemperatur liegt zwischen 50 und 180, vorzugsweise zwischen 70 und 150 und insbesondere im Bereich von 90 bis 150°C. Man läßt sodann unter Einhaltung der gewählten Reaktionstemperatur noch 5 bis 50, vorzugsweise 10 bis 20 Stunden nachreagieren.

Unter Alkalibasen sollen Alkalimetallhydroxide wie Natriumhydroxid, Kaliumhydroxid, Lithiumhydroxid und Alkalialkoholate wie z.B. Natriummethylat, Natriumethylat, Kaliumisopropylat und Kaliumtertiärbutylat verstanden werden. Geeignete Lösungsmitel sind bevorzugt Methanol, Ethanol, Isopropanol, Butanol und Hexanol.

Der erzielbare Polymerisationsgrad hängt ab von der Temperatur und der Basizität des Katalysators. Je höher die Temperatur und die Basizität des Katalysators ist, umso höher ist auch der Polymerisationsgrad.

Die Verbindungen gemäß Formel (II) mit X = Wasserstoff und Y = Hydroxyl werden durch Reduktion derjenigen, in denen X und Y zusammen Sauerstoff bedeuten, nach literaturbekannten Hydriermethoden [H. Hörmann, Angew. Chem. 68 (1956), 601, E. Schenker, Angew. Chem. 73 (1961), 81, W. G. Brown, Org. Reactions 6 (1951), 469—510, Houben-Weyl, Bd. IV/2, S. 303—312, 318—328 (1955)] erhalten.

Durch unvollständiges Hydrieren lassen sich auch Polyalkylpiperidinpolymerisate herstellen, die Keto- und Hydroxygruppen im gleichen Molekül erhalten. Die Hydrierung wird z.B. so vorgenommen, daß man das ketogruppenhaltige Polyalkylpiperidinpolymerisat in der 1,5- bis 10fachen Gewichtsmenge absoluten Ethanols löst, mit 1 bis 2,0 Äquivalenten NaBH versetzt und bei Raumtemperatur 20 bis 60 Stunden rührt. Anschließend gießt man zur Hydrolyse in die 5- bis 10fache Menge Wasser und nutscht das Hydrierungsprodukt ab. Sowohl die nicht hydrierten als auch die hydrierten Polymerisate können durch Umfällen gereinigt werden.

Die neuen Polyalkylpiperidinpolymerisate sind schwer flüchtige Verbindungen, die, insbesondere in Form der Hydrierungsprodukte, sehr gute Lichtschutzmittel für synthetische Polymere darstellen; sie dienen jedoch in erster Linie zur Herstellung anderer hochwertiger Kunststoffstabilisatoren.

Die erfindungsgemäßen Verbindungen sind, wie bereits erwähnt, durch die Kombination stabilisierende Wirkung/Schwerflüchtigkeit von erheblicher technischer Bedeutung. In vielen Anwendungsgebieten genügen nämlich die bisher bekannten Lichtschutzmittel nicht den technischen Anforderungen. Die meist niedrigmolekularen Verbindungen sind bei den hohen Verarbeitungstemperaturen von oft mehr als 250°C stark flüchtig. Die Folge davon sind Wirksamkeitsminderung durch Stabilisatorverluste und Belästigung bei der Verarbeitung durch Dämpfe. Auch polymere Stabilisatoren können diese Nachteile besitzen, wenn die Polymerketten bei den Verarbeitungstemperaturen des zu stabilisierenden synthetischen Polymeren zur Crackung neigen. Überraschenderweise besitzen die neuen Polyalkylpiperidinpolymerisate alle diese Nachteile nicht und sind trotz der Häufung polarer Gruppen sehr gut mit den zu stabilisierenden synthetishen Polymeren verträglich.

Die Produkte gemäß der Erfindung eignen sich zum Stabilisieren von halogenfreien und halogenhaltigen Homo- und Copolymeren. Im einzelnen seien genannt: Homopolymerisate von Olefinen, Dienen und Styrol, wie z.B. Polyethylen niedriger und hoher Dichte, Polypropylen, Polystyrol, Polybutadien und Polyisopren, Copolymere von Olefinen, Dienen und Styrol miteinander oder anderen olefinisch ungesättigten Monomeren, wie Ethylen-Propylen-Copolymere, Ethylen-Buten-Copolymere, Styrol-Butadien-Copolymere, Ethylen-Vinylacetat-Copolymere und Acrylnitril-Butadien-Styrol-Copolymere, Homopolymere von Vinylchlorid und Vinylidenchlorid und Copolymere dieser Monomeren untereinander und mit anderen olefinisch ungesättigten Monomeren. Des weiteren sollen auch Polyurethane, Polyacetale, Polyester, Polyamide, Polyacrylate und Epoxydharze eingeschlossen sein. Bevorzugt sind Poly-$\alpha$-Olefine wie Polyethylene und insbesondere Polypropylene.

Die neuen stabilisierenden Polymeren werden nach allgemein üblichen Methoden in die Polymermassen eingearbeitet. Alternativ kann man auch eine Lösung, Suspension oder Emulsion des Stabilisators mit dem Polymeren direkt oder mit einer Lösung, Suspension oder Emulsion desselben vermischen und das Lösungsmittel anschließend entfernen.

Die erfindungsgemäßen Stabilisatoren sind für sich allein oder im Gemisch mit einem oder mehreren der bei der Kunststoffverarbeitung üblichen Stabilisatoren, wie z.B. Antioxidantien auf Phenol- und Sulfidbasis, UV-Absorbern und Lichtschutzmitteln, Phosphitstabilisatoren, Metallverbindungen, Epoxystabilisatoren und mehrwertigen Alkoholen einsetzbar. In den zu stabilisierenden Kunststoffmassen können ferner Flammschutzmittel und Pigmente, Farbstoffe, Antistatika und Füllstoffe, wie z.B. Glasfasern, anwesend sein.

Beispiele für geeignete Antioxidantien sind solche vom Typ der sterisch gehindedrten Phenole wie

2,6-Di-tert.-butyl-p-kresol, 2,6-Di-octadecyl-p-kresol, 4,4'-Butyliden-bis-(2,6-di-tert.-butyl-phenol)-4,4'-Thio-bis(2-tert.-butyl-5-methylphenol), phenolische Triazinverbindungen, Thiodipropionsäureester von Fettalkoholen, Dioctadecylsulfid und -disulfid.

Zu den UV-Absorbern und Lichtschutzmitteln gehören z.B. 2-(2'-Hydroxyphenyl)-benztriazole wie 2-(2'-Hydroxy-5'-methyl-phenyl)-benztriazol, 2-Hydroxybenzophenone wie 2-Hydroxy-4-octoxy-benzophenon, Stabilisatoren aus der Gruppe der Salizylate wie Octylphenylsalizylat, Nickelchelate, Oxalsäurediamide und sterisch gehinderte Piperidinverbindungen.

Als Phosphite sind Trisnonylphenylphosphit, Trislaurylphosphit oder auch Ester des Pentaerythritphosphits zu nennen.

Unter als Stabilisatoren bekannten Metallverbindungen werden verstanden: Calcium-, Barium-, Strontium-, Zink-, Cadmium-, Magnesium-, Aluminium- und Bleiseifen aliphatischer Carbonsäuren oder Oxycarbonsäuren mit etwa 12 bis 32 C-Atomen, Salze der genannten Metalle mit aromatischen Carbonsäuren wie Benzoate oder Salizylate sowie (Alkyl-)Phenolate dieser Metalle, ferner Organozinnverbindungen, wie z.B. Dialkylzinnthioglykolate und Carboxylate.

Bekannte Epoxystabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d.h. bevorzugt Alkohole mit 5 oder 6 C-Atomen und 3 bis 6 OH-Gruppen.

Eine wirksame Stabilisatorkombination für Poly-$\alpha$-Olefine, wie z.B. Hoch-, Mittel- und Niederdruckpolymerisate von $C_2$- bis $C_4$-$\alpha$-Olefinen, insbesondere Polyethylen und Polypropylen oder von Copolymerisaten derartiger $\alpha$-Olefine besteht, bezogen auf 100 Gewichtsteile Polymer, beispielsweise aus 0,01 bis 5 Gewichtsteilen einer der erfindungsgemäß zu verwendenden Stabilisatoren, gegebenenfalls 0,01 bis 4 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat sowie gegebenenfalls 0,1 bis 5 Gewichtsteilen eines Phosphits und gegebenenfalls 0,01 bis 5 Gewichtsteilen eines bekannten UV-Stabilisators aus der Gruppe der Alkoxyhydroxybenzophenone, 4-Hydroxyphenylbenzotriazole, Benzylidenmalonsäuremononitrilester oder der sogenannten Quencher wie z.B. Nickelchelate.

Die folgenden Beispiele dienen der näheren Erläuterung der Erfindung. Die Molgewichte der polymeren Ketone lassen sich aus den polymeren Reduktionsprodukten ableiten.

### Beispiel 1a

### Di-2,2,6,6-tetramethylpiperidin-4-on-3-methan

In einer 500-ml-Rührapparatur werden 155 g (1 Mol) 2,2,6,6-Tetramethylpiperidin-4-on- in 50 ml Methanol vorgelegt. Dazu wird innerhalb von 5 Stunden bei 60 bis 70°C eine Lösung von 15 g (0,5 Mol) Paraformaldehyd, 2 g KOH und 80 g Methanol getropft. Hierauf wird 25 Stunden bei derselben Temperatur nachgerührt. Anschließend wird zunächst im Vakuum das Lösungsmittel abgezogen, worauf man den Rückstand im Vakuum destilliert, wobei nach dem Abdestillieren von nicht umgesetztem 2,2,6,6-Tetramethylpiperidin-4-on die Titelverbindung als rotes Öl gewonnen wird.

$K_p$ 0,4 mbar 180°C; Ausbeute 40 g; Molgewicht 330

### Beispiel 1b

### Reduktion des Ketons gemäß Beispiel 1a

20 g (0,062 Mol) der Verbindung gemäß Beispiel 1a werden in 100 ml absolutem Ether gelöst und zu einer Suspension aus 8 g (0,21 Mol) Lithiumaluminiumhydrid und 100 ml absolutem Ether getropft. Anschließend wird 10 Stunden am Rückfluß gekocht, mit 20 ml Wasser unter Kühlung versetzt sind nach dem Abtrennen des Ethers der Feststoff mehrfach mit heißem Heptan extrahiert.- Beim Einengen des Extraktes kristallisiert Di-2,2,6,6-tetramethylpiperidin-4-on-3-methan aus, das durch Filtration gewonnen wird.

Weißer Feststoff; Fp. 195 bis 198°C

### Beispiel 2a

### Darstellung eines polymeren Ketons

Die Umsetzung und Aufarbeitung erfolgt analog Beispiel 1a, jedoch wird als Katalysator 1 g Natriummethylat anstelle von KOH verwendet und 26 Stunden nachgerührt. Nach dem Abdestillieren von ca. 90 g nicht umgesetztem 2,2,6,6-Tetramethylpiperidon bleiben 56 g eines spröden, dunkelroten Harzes zurück.

### Beispiel 2b

### Reduktion des polymeren Ketons

Das polymere Keton wird in 200 ml absolutem Ethanol gelöst, mit 26,5 g Natriumborhydrid versetzt und 48 Stunden bei Raumtemperatur gerührt. Danach wird das Reaktionsgemisch in 2 Liter Wasser eingerührt und leicht erwärmt. Nach Beendigung der Wasserstoffentwicklung wird abgenutscht, mit Methanol/Wasser umgefällt und getrocknet. Gelbes Pulver; Erweichungspunkt 103°C; Molgewicht 3960.

Beispiel 3a

Darstellung eines oligomeren Ketons

In einer 500-ml-Rührapparatur werden 31 g (0,2 Mol) 2,2,6,6-Tetramethylpiperidin-4-on, 6 g (0,2 Mol) Paraformaldehyd, 0,5 g KOH-Pulver und 200 ml n-Butanol 65 Stunden unter Rückfluß gekocht. Nach dem Abziehen des Lösungsmittels bleibt in quantitativer Ausbeute ein dunkelrotes Harz.

Beispiel 3b

Reduktion des oligomeren Ketons

37 g des oligomeren Ketons werden in 200 ml Ethanol gelöst, mit 3,8 g Natriumborhydrid versetzt und 48 Stunden bei Raumtemperatur gerührt. Dann werden ca. 100 ml Ethanol abdestilliert und der Rückstand mit 500 ml Wasser verrührt (3 Stunden), hierauf wird abgenutscht und aus Methanol/Wasser umgefällt, wobei 33 g eines gelben Produktes vom Fp. 79 bis 81 °C zurückbleiben. Molgewicht 690.

Beispiel 4a

Darstellung eines oligomeren Ketons

Die Darstellung und Aufarbeitung erfolgt analog Beispiel 3a in n-Hexanol als Lösungsmittel, wobei ebenfalls ein dunkelrotes Harz zurückbleibt.

Beispiel 4b

Reduktion des oligomeren Ketons

Die Umsetzung und Aufarbeitung erfolgt analog Beispiel 3b mit denselben Gewichtsmenge, wobei 28 g eines Produktes mit Fp. 99 bis 102°C und Molgewicht 670 anfallen.

Beispiel 5a

Darstellung eines oligomeren Ketons

In einem 2-Liter-Stahlautoklaven werden 155 g (1 Mol) 2,2,6,6-Tetramethylpiperidin-4-on, 30 g (1 Mol) Paraformaldehyd und 2 g KOH in 400 g Methanol gelöst und 7 Stunden auf 120°C erhitzt. Anschließend wird das Lösungsmittel abgezogen, wobei ein dunkelrotes Harz zurückbleibt.

Beispiel 5b

Reduktion des oligomeren Ketons

Das Harz wird in 500 ml absolutem Ethanol gelöst, mit 35 g Natriumborhydrid versetzt und 48 Stunden bei Raumtemperatur gerührt. Das Reaktionsgemisch wird in 2 Liter Wasser gegeben und 2 Stunden gerührt. Der ausgefallene Feststoff wird abgenutscht, mit 500 ml handwarmem Wasser verrührt, abgenutscht und getrocknet.

85 g eines gelben Pulvers, Fp. 153°C, Molgewicht 960

Beispiel 6a

Darstellung eines polymeren Ketons

Die Darstellung erfolgt analog Beispiel 5a, jedoch mit 5 g NaOH anstelle von KOH und einer Reaktionsdauer von 20 Stunden. Ein Teil der Reaktionslösung wird aufgearbeitet, wobei man ein rotes Harz erhält.

Beispiel 6b

Katalytische Hydrierung des polymeren Ketons

Zu dem Rest der nach Beispiel 6a erhaltenen Reaktionslösung werden 7,0 g Eisessig zur Neutralisation der NaOH und 30 g Raney-Nickel gegeben. Dann wird bei 100°C und einem Wasserstoffdruck von 150 bar hydriert. Die Wasserstoffaufnahme ist nach 5 Stunden beendet. Die Reaktionslösung wird abgenutscht, wobei das Raney-Nickel wiedergewonnen wird. Das Filtrat rührt man in 3 Liter Wasser ein. Hierbei fällt der gebildete Polyalkohol aus und wird durch Filtration gewonnen.

Molgewicht 1000, Fp. 130 bis 135°C.

Durch Lösen in Ether und Ausfällen mit Petrolether kann eine Fraktion vom Molgewicht 1360 (Fp. 160 bis 170°C) gewonen werden.

Beispiel 7

Darstellung eines polymeren Ketons

In eine Mischung aus 155 g (1 Mol) 2,2,6,6-Tetramethylpiperidon und 200 ml Hexanol wird bei 130°C eine Lösung aus 30 g (1 Mol) Paraformaldehyd, 200 ml Methanol und 7,5 g Natriummethylat innerhalb von 5 Stunden zugetropft. Das Methanol destilliert laufend ab. Es wird 20 Stunden bei 130°C nachgerührt, mit Eisessig neutralisiert, das Natriumacetat abgenutscht und im Vakuum bei 120°C eingedampft. Beim Abkühlen bleibt ein sprödes, orangerotes Harz zurück. Ausbeute 140 g = 83% d. Th., Fp. 130 bis 135°C, Molgewicht 690.

Beispiel 8

Dieses Beispiel zeigt die lichtstabilisierende Wirkung der erfindungsgemäßen Verbindungen beim Einsatz in einem Poly-$\alpha$-Olefin.

100 Gewichtsteile Polypropylen mit einem Schmelzindex $i_5$ von ca. 6 g/10 min. (bestimmt nach ASTM D 1238—62 T) und einer Dichte von 0,90 wurden mit

0,1 Gew.-Teilen Pentaerythrityl-tetrakis-3-(3,5-di-tert.-butyl-4-hydroxyphenyl)-propionat,

0,2 Gew.-Teilen Calciumstearat und

0,1 Gew.-Teilen des zu prüfenden erfindungsgemäßen Stabilisators vermischt.

Um eine möglichst gleichmäßige Verteilung auf dem Polymerkorn zu erreichen, wurden die Stabilisatoren in einem Lösungsmittel gelöst, und die Lösung wurde unter Rühren in das Polypropylenpulver eingetropft, wobei durch gleichzeitige Bestrahlung mit einer IR-Lampe der größte Teil des Lösemittels wieder abdampfte. Nach ca. 20 Minuten wurde das Calciumstearat hinzugegeben und noch weitere 10 Minuten gemischt. Lösemittelreste wurden durch Trocknen bei 50°C/120 Min. im Trockenschrank entfernt. Daraus wurden auf einer Windsor-Spritzgußmaschine der Type SP 50 bei 250°C 60 x 60 x 1 mm-Platten verspritzt. Aus diesen Platten wurden Prüfkörper nach DIN 53 455 Form 3, verkleinert im Maßstab 1:3, ausgestanzt. Die als Vergleichsmuster benötigten Prüfkörper wurden analog, jedoch unter Fortlassen des zu testenden Stabilisators (Versuch a) bzw. unter Verwendung bekannter Lichtstabilisatoren (Versuche b und c) hergestellt.

Zur Bestimmung der Lichtbeständigkeit wurden die Proben in einer Xenotest-1200-Apparatur der Firma Original Hanau Quarzlampen GmbH der Bestrahlung mit Wechsellicht unterworfen. Die Strahlungsintensität wurde durch UV-Filter (Spezialfilterglas d = 1,7 mm) moduliert. Die Lichtbeständigkeit wurde nach DIN 53 387 (17 Min. befeuchten, 3 Min. beregnen, Schwarztafeltemperatur 45°C, Luftfeuchtigkeit 70 bis 75%) geprüft. Gemessen wurde die Belichtungszeit in Stunden und die Reißdehnung auf einer Zugprüfmaschine der Firma Instron bei einer Abzugsgeschwindigkeit von 5 cm/Min. bestimmt.

Die Ergebnisse sind in der nachfolgenden Tabelle zusammengestellt.

| Versuchs-Nr. | Stabilisator nach Beispiel | Belichtungszeit |
|---|---|---|
| a) | Kontrolle (ohne Stabilisator) | <345 |
| b) | Benzophenon-Stabilisator[1)] | <950 |
| c) | HALS-Stabilisator[2)] | 950 |
| d) | 2 b | >1000 |
| e) | 5 b | >1000 |

1) = 2-Hydroxy-4-n-octyloxybenzophenon

2) = Bis-2,2,6,6-tetramethylpiperidyl-sebacat

**Patentansprüche**

1. Verfahren zur Herstellung von Polyalkylpiperidin-Formaldehyd-Polymerisaten durch Umsetzen eines Polyalkylpiperidons-4 der allgemeinen Formel (I)

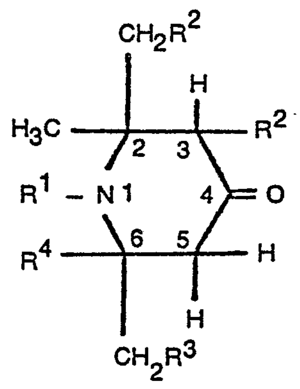

(I)

in welcher

$R^1$ Wasserstoff, Sauerstoff oder $C_1$- bis $C_{12}$-Alkyl bedeutet,

$R^2$ und $R^3$ entweder gleich sind und Wasserstoff oder eine $C_1$- bis $C_5$-Alkylgruppe sind, wobei dann $R^4$ eine Methylgruppe ist, oder auch

$R^2$ Wasserstoff oder $C_1$- bis $C_5$-Alkyl ist, und

$R^3$ und $R^4$ zusammen mit den an sie gebundenen Kohlenstoffatomen eine $C_5$- oder $C_6$-Cycloalkylgruppe oder eine Gruppe der Formel

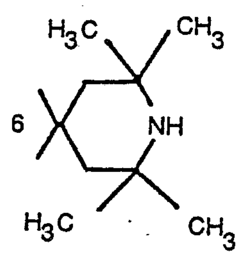

bedeuten,

mit Paraformaldehyd in Gegenwart eines alkalischen Katalysators und eines aliphatischen $C_1$- bis $C_{10}$-Monoalkohols bei erhöhten Temperaturen, wobei die Umsetzungsprodukte nach an sich bekannten Verfahren auch noch vollständig oder teilweise hydriert werden können, dadurch gekennzeichnet, daß man die 0,5- bis 1,1fach molare Menge Paraformaldehyd, bezogen auf Polyalkylpiperidon, bei Anwesenheit einer Alkalibase einsetzt und bei 50 bis 180 $\alpha$ arbeitet.

2. Polyalkylpiperidin-Formaldehyd-Polymerisate mit Polymerisationsgraden von 2 bis 200 und ihre Hydrierungs- oder Teilhydrierungsprodukte, erhalten nach Anspruch 1.

3. Verwendung der Polyalkylpiperidinpolymerisate nach Anspruch 2 zum Stabilisieren von synthetischen Polymeren.

**Revendications**

1. Procédé de préparation de polymères polyalkyl-pipéridine/formaldéhyde par réaction, avec le paraformaldéhyde, d'une polyalkyl-pipéridone-4 répondant à la formule générale I

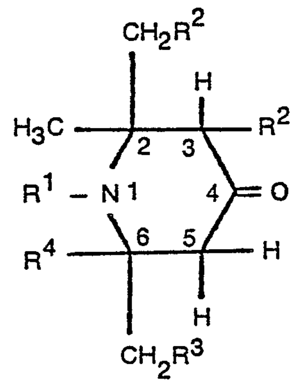

(I)

dans laquelle

$R_1$ représente l'hydrogène, l'oxygène ou un alkyle en $C_1$—$C_{12}$, — ou bien

$R_2$ et $R_3$ sont identiques et représentent chacun l'hydrogène ou un alkyle en $C_1$—$C_5$, auquel cas $R_4$ représente un méthyle, — ou bien

$R_2$ représente l'hydrogène ou un alkyle en $C_1$—$C_5$ et

$R_3$ et $R_4$ forment ensemble et avec les atomes de carbone auxquels ils sont liés un radical cycloalkyle en $C_5$ ou $C_6$ ou un radical de formule:

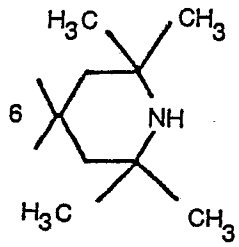

en présence d'un catalyseur alcalin et d'un mono-alcool aliphatique en $C_1$—$C_{10}$, à des températures élevées, les produits réactionnels pouvant en plus être hydrogénés, totalement ou partiellement, par des méthodes connues, procédé caractérisé en ce qu'on met en jeu de 0,5 à 1,1 fois la quantité molaire de paraformaldéhyde, par rapport à la polyalkyl-pipéridone, en présence d'une base alcaline, et on opère à une température de 50 à 180°C.

2. Polymères polyalkyl-pipéridine/formaldéhyde à degrés de polymérisation compris entre 2 et 200 et leurs produits d'hydrogénation partielle ou totale qui ont été obtenus selon la revendication 1.

3. Application des polymères polyalkyl-pipéridiniques selon la revendication 2 à la stabilisation de polymères synthétiques.

## Claims

1. A process for the preparation of polyalkyl-piperidine/formaldehyde polymers by reacting a polyalkyl-piperidone-4 of the formula (I)

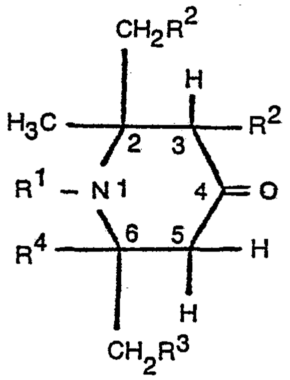

(I)

in which

$R^1$ is hydrogen, oxygen or $C_1$—$C_{12}$-alkyl,

$R^2$ and $R^3$ are either identical and represent hydrogen or a $C_1$—$C_5$-alkyl group,

$R^4$ being a methyl group in this case; or

$R^2$ is hydrogen or $C_1$—$C_5$-alkyl, and

$R^3$ and $R^4$ together with the carbon atoms linked to them form a $C_5$ or $C_6$ cycloalkyl group, or a group of the formula

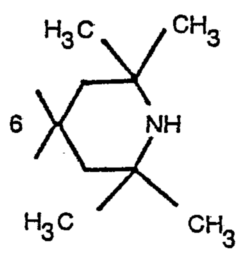

with paraformaldehyde in the presence of an alkaline catalyst and of an aliphatic $C_1$- to $C_{10}$-monoalcohol at elevated temperature, the products thus obtained being optionally hydrogenated either completely or partially according to known processes, which comprises inserting the 0,5- to 1,1-fold molar amount of paraformaldehyde, calculated on the polyalkylpiperidone, in the presence of an alkaline base and reacting at a temperature of from 50 to 180°C.

2. Polyalkyl-piperidine/formaldehyde polymers of a polymerization degree of from 2 to 200 and the hydrogenation or partial hydrogenation products thereof, obtained as claimed in claim 1.

3. Use of the polyalkylpiperidine/formaldehyde polymers as claimed in claim 2 for stabilizing synthetic polymers.